# EUROPEAN PATENT SPECIFICATION

(11) **EP 2 398 344 B1**
(45) Date of publication and mention of the grant of the patent: **13.07.2016**
(21) Application number: 10711722.8
(22) Date of filing: 17.02.2010
(51) Int. Cl.: A23L 33/17, A23L 33/00, A23L 5/00

(54) **FOOD CONTAINING PROLACTIN**
PROLAKTIN ENTHALTENDE NAHRUNGSMITTEL
ALIMENT CONTENANT DE LA PROLACTINE

(30) Priority: 17.02.2009 US 153080 P
(43) Date of publication of application: 28.12.2011
(73) Proprietor: Technion Research & Development Foundation Ltd., 32000 Haifa (IL)
(72) Inventor: SHEHADEH, Naim, 24908 Kfar Yasif (IL)
(74) Representative: Becker Kurig Straus
(86) International application number: PCT/IL2010/000139
(87) International publication number: WO 2010/095130

(56) References cited:
- WO-A1-91/05548
- WO-A2-03/044174
- WO-A2-2005/115473
- US-A- 5 162 303
- US-A1- 2006 127 453

## Description

### FIELD OF THE INVENTION

This invention relates to the use of prolactin for the manufacture of a nutritional composition and its application in supplementing the diet of mammals.

### BACKGROUND OF THE INVENTION

One of the major effects of breast-feeding is the support of gut maturation. Breast-fed milk may also influence small intestinal growth and functional maturation.

Prolactin has been detected in milk of the cow (Erb et al., 1977), goat (McMurty and Malven, 1974), Pig (Mulloy and Malven, 1979) and primates (Gala et al., 1975).

Prolactin is a polypeptide hormone that is now appreciated to have over 300 separate biological activities, including promoting lactation in mammals, and roles in reproduction and homeostasis (Freeman et al., 2000).

Prolactin may also be an important agent in the development of neuroendocrine, reproductive and immune function in neonatal mice and rats (Sinha and Vanderlaan, 1982, Grove et al., 1991). Moreover, prolactin in human milk may have a physiological significance for newborn humans, including enhancing calcium absorption across intestinal epithelial membranes (Taketani and Mizuno, 1985, Amnattananakul et al., 2005).

Prolactin receptors (long and short forms) were identified in the epithelial cells of rat duodenum, jejunum, ileum and colon (Ouhtit et al., 1994). In humans, duodenal enterocytes also express prolactin receptors and prolactin receptors are expressed in villous columnar epithelial cells of the duodenum from 12 to 14 weeks of gestation (Freemark et al., 1997). Indeed, prolactin has been found to be absorbed in the intestine of neonates (Amnattananakul et al., 2005). Therefore, the small intestine in newborn humans may be a direct target organ of prolactin.

However, human neonates and newborn animal infants are frequently weaned of their natural food or feed immediately or shortly after birth, and are then nourished primarily with artificially produced food substitutes.

A putative alternative exogenous source of prolactin is unprocessed milk and eggs. However, most industrial food or feed production processes involve manufacturing conditions that are destructive to the viability of prolactin. In addition, supply chain constraints impose longer shelf life requirements such that extended storage under adverse conditions is enabled. Unfortunately, such requirements often result in loss of efficacy of the biological activity of such compound. Indeed, we have shown that prolactin concentration is significantly higher in fresh human milk (329.5±268.5mU/L) compared with pasteurized human milk (120.8±103 mU/L). In addition, only trace amounts of prolactin were detected in commercial bovine fresh milk and in infant formulas (Vaxman and Shehadeh, 2008).

Food components destroyed during processing of food may be extracted from plants, recombinant organisms or otherwise artificially generated, and may be produced at a lower cost, be free of bacteria and viruses frequently found in traditional sources, and be better accepted as healthier and safer by both regulatory authorities and the general public. The technology for production of human DNA recombinant prolactin is available (Price et al., 1995).

Although a source of prolactin for newborn babies is mother's milk, and despite evidence indicating that this source of prolactin may have a physiological importance (Taketani and Mizuno, 1985), it is nevertheless at present not clear whether prolactin should be added to food preparations for babies. For example, in suckling rats only 16 % of the total plasma prolactin originates from mother's milk (Gonella et al., 1989; Whitworth and Grosvenor, 1995). In addition, angiogenesis, the development of blood vessels, has been found to be inhibited by proteolytic fragments of native prolactin (Clapp C and De La Escalera GM, Prolactins: novel regulators of angiogenesis, News Physiol Sci 12: 231-237, 1997).

A further factor complicating the decision of whether, and also how, to add prolactin to food stuffs is that there are many variants of prolactin (http://en.wikipedia.org/wiki/Prolactin; Freeman, 2000).

Due to the great variance, bioassays and immunoassays can give different results due to differing glycolyzation, phosphorylation sulfation, and deamidation and degradation extents. The prolactin molecules may result from alternative splicing of the primary transcript, proteolytic cleavage and other post-translational modifications of the amino acid chain, and prolactin molecules may interact between themselves to produce dimer and polymer prolactin or with other proteins such as immunoglobulins. The roles of these variants are unclear.

Therefore, there is at present uncertainty as to which form, if any, of prolactin should be provided in baby foods, and how to prevent its degradation during preparation and storage.

International Application publication No. WO 2003/044174 discloses colostrum associated mammalian Serum Amyloid A (SAA) promoter sequences. The promoters can be used in transgenic protocols for tissue specific expression and expression constructs, vectors and host cells are disclosed. Also the regulatory features of these promoters in colostrum associated SAA productions are exploited to treat disease states associated with or influenced by colostrum associated SAA production.

International Application publication No. WO 2005/115473 discloses means for protecting and incorporating bioactive compounds, insulin or derivative thereof serving as examples, in food or feed formulations used to enhance the health status and growth performance of human and non-human organisms.

Despite reservations regarding adding prolactin to food, we have recognized a need for the addition and have devised a nutritional feed composition that will answer the need for optimal nutrition to newborn humans, capable of delivering biomaterials including prolactin in a manner that will guarantee its viability to the infant, as well as along the supply chain of its manufacture and similar need is recognized in supplementing the nutritional formula of term and preterm human neonates.

### SUMMARY OF THE INVENTION

The present invention provides the use of prolactin for the manufacture of a nutritional composition for enhancing small intestinal growth, small intestinal functional maturation and/or calcium absorption across intestinal epithelial membranes enhancing gut maturation, and/or stimulating mitosis in T lymphocytes. Said composition comprises prolactin identical or similar or analogous to prolactin found in a natural food source microencapsulated in at least one protective layer. Release of said prolactin from the protective layer in said subject is the result of an environmental trigger.

According to one embodiment, the natural food source is selected from the group consisting of natural unprocessed milk, natural unprocessed eggs, animal tissue, colostrum and serum.

According to another embodiment, the prolactin comprises at least one prolactin variant, each variant being an analogue of a naturally occurring prolactin or a functional derivative thereof.

According to another embodiment, the subject is a mammal, preferably a human.

According to yet another embodiment, the nutritional composition is specifically formulated for said subject. The subject can be a mammal and the at least one variant in the composition may be homologous to a variant of the mammal. Further, all variants in the composition may be homologous to a variant in the mammal. Alternatively, the at least one variant in the composition is identical to a variant in the mammal, preferably wherein all of the variants in the composition are identical to a variant in the mammal.

According to a preferred embodiment, the mammal is a human, preferably wherein the human is selected from the group consisting of a neonate, a preterm human infant, a term human infant, a baby, a toddler, an adolescent, an adult and an old person.

According to a further embodiment, the prolactin comprises a 23 kDa form.

According to yet another embodiment, the prolactin comprises at least one prolactin variant, each variant being an analogue of a naturally occurring prolactin or a functional derivative thereof, wherein the at least one variant is selected from the group consisting of non-glycosylated prolactin, non-phosphorylated prolactin, prolactin monomer and any combination thereof.

According to another embodiment, the composition comprises at least one complex between the prolactin and the at least one protective layer. Preferably wherein the composition further comprises one or more water-soluble polymer selected from the group polyethylene glycol, copolymers of polyethylene glycol and polypropylene glycol, carboxymethyl cellulose, dextran, polyvinyl alcohol, polyvinylpyrrolidone and polyproline, for complexation between the prolactin and at least one protective layer via covalent attachment of the water-soluble polymers to both the prolactin and the protective layer. Even more preferably, the protective layer is specifically designed to degrade as a response to the environmental trigger, wherein said environmental trigger is a change in at least one of temperature, moisture content, pressure, pH, ionic strength, enzymatic activity, time passed since providing the subject with the nutritional composition, or any combination thereof, preferably wherein the protective layer is selected from the group consisting of polysaccharide, maltodextrin, milk powder, whey protein, lipid, gum, celluloses, amorphous lactose, or combinations thereof.

The present invention further provides the use of prolactin for the manufacture of a supplemented nutritional food or feed or drink for enhancing small intestinal growth, small intestinal functional maturation and/or calcium absorption across intestinal epithelial membranes enhancing gut maturation, and/or stimulating mitosis in T lymphocytes. The supplemented nutritional food comprises a nutritional food and a prolactin composition, the prolactin composition comprising prolactin identical or similar or analogous to prolactin found in a natural food source microencapsulated in at least one protective layer. Release of said prolactin from the at least one protective layer in said subject is the result of an environmental trigger.

According to one embodiment, the manufacture comprises adding the prolactin composition to the nutritional food or feed or drink. Alternatively or preferably the manufacture comprises:
a. preparing a protected prolactin comprising mixing said prolactin with an appropriate first protective material forming a first blend;
b. processing the first blend to form a dry second blend comprising prolactin in at least one protective layer, wherein said protective layer is specifically designed to degrade as a response to change in an environmental trigger; and
c. adding the dry second blend to said nutritional food or nutritional feed or drink.

The preparing a protected prolactin may further comprise:
a. forming a round core from the second blend;
b. drying the core;
c. collecting the dehydrated core;
d. making a first suspension, comprising the dehydrated core in a liquid second protective material;
e. drying the first suspension in a fluidized bed drier;
f. collecting the fluidized bed -dried suspension;
g. making a second suspension, comprising the collected dried suspension in a liquid third protective material; and
h. drying the second suspension in a fluidized bed drier. Preferably, the first blend is liquid.

Also preferably, the first suspension further comprises at least one of a Maltodextrin, a vitamin, an antioxidant, a protease inhibitor, a growth hormone, an EGF (Epidermal Growth Factor), an insulin and insulin- like growth factor, an insulin-like growth factor's binding protein, an immunoglobulin, a proline-rich polypeptide, a lactoferrin, a protease, a lactalbumin, an interleukin, a lysozyme, a TGFA (Transforming Growth Factor A), a PDGF (Platelet Derived Growth Factor) or a combination thereof.

Also preferably, the second suspension further comprises at least one of a maltodextrin, a vitamin, an antioxidant, a protease inhibitor, a growth hormone, an EGF (Epidermal Growth Factor), an insulin and insulin- like growth factor, an insulin-like growth factor's binding protein, an immunoglobulins, a proline-rich polypeptide, a lactoferrin, a protease, a lactalbumin, an interleukin, a lysozyme, a TGFA (Transforming Growth Factor A), a PDGF (Platelet Derived Growth Factor) or a combination thereof.

According to a preferred embodiment, the maltodextrin has a dextrose equivalent (DE) between 2 and 64, preferably wherein the dextrose equivalent is 18.

According to another embodiment, the prolactin is derivatized ex- vivo, preferably wherein the derivatization is done by at least one of enzymatic digestion, physical methods, chemical methods, or any combination thereof. Alternatively or preferably, the use further comprises an agglomeration step, preferably wherein the agglomeration step results in particle average diameter between about 0.1 and about 5,000 micrometers.

According to a preferred embodiment, preparing a protected prolactin further comprises:
a. forming a round core from the second blend;
b. drying the core;
c. collecting the dehydrated core;
d. making a first suspension, comprising the dehydrated core in a liquid second protective material;
e. drying the first suspension in a fluidized bed drier;
f. collecting the fluidized bed -dried suspension;
g. making a second suspension, comprising the collected dried suspension in a liquid third protective material; and
h. drying the second suspension in a fluidized bed drier.

The core may be inert, and/or forming the round core further comprises:
a. flash freezing said liquid blend;
b. collecting the droplets produced;
c. lyophilizing the collected droplets; and
d. collecting the lyophilized droplets, thereby creating a round core.
According to preferred embodiments, the core may comprise prolactin, or the third protective material is designed to thermally protect prolactin for at least 2 minutes at a temperature of no less than 95°C, or the second protective material is designed to protect said prolactin from proteolytic enzymes and pH of no more than 4.75. Also disclosed but not forming part of the present invention is a method for preparing protected prolactin in a nutritional food or feed or drink is provided, the method comprising:
preparing a protected prolactin comprising:
   mixing said prolactin with a first protective material forming a first blend;
   processing the first blend to form a dry second blend comprising at least one protective layer,
   wherein said protective layers are specifically designed to degrade as a response to change in an environmental trigger, and
   adding the protected prolactin to said nutritional food or nutritional feed or drink.

A supplemented nutritional food or feed or drink of a mammal, may comprise any one of said compositions, incorporated in said nutritional food or feed or drink, the composition thereby being a supplement to said food or feed or drink.

The supplemented nutritional drink may have a level of prolactin between 40 and 800 mU/L. Preferably, the supplemented nutritional drink has a level of prolactin between 100 and 600 mU/L.

In the supplemented nutritional food the food may be selected from baby or newborn or infant formulas, dried milk, and milk substitute.

In the supplemented nutritional food the formula may be a dry powder and the prolactin composition may be dry.

Alternatively, the baby formula is a liquid prepared from a dry powder.

Preferably, the prolactin composition is evenly distributed in the food.

Prolactin may also be provided for manufacture of a nutritional composition for enhancing mall intestinal growth, small intestinal functional maturation and/or calcium absorption across intestinal epithelial membranes enhancing gut maturation, and/or stimulating mitosis in T lymphocytes, and/or increasing rate of weight gain, and/or improving the FCR, and/or reducing incidence of diarrhea and/or other gastric disorders and/or increasing the life expectancy of a subject, the composition comprising prolactin identical or similar or analogous to prolactin found in a natural food source, and at least one protective layer, wherein release of said prolactin from the composition in said subject is the result of an environmental event.

Prolactin may also be provided for manufacture of a supplemented nutritional food or feed or drink for enhancing small intestinal growth, small intestinal functional maturation and/or calcium absorption across intestinal epithelial membranes enhancing gut maturation, and/or stimulating mitosis in T lymphocytes, and/or increasing rate of weight gain, and/or improving the FCR, and/or reducing incidence of diarrhea and/or other gastric disorders and/or increasing the life expectancy of a subject, the supplemented nutritional food comprising nutritional food and a prolactin composition, the prolactin composition comprising prolactin identical or similar or analogous to prolactin found in a natural food source, and at least one protective layer, wherein release of said prolactin from the composition in said subject is the result of an environmental event.
The supplemented nutritional food or feed or drink manufacture may comprise adding said prolactin composition to said nutritional food or feed or drink

The manufacture may comprise:
preparing a protected prolactin comprising:
   mixing said prolactin with an appropriate first protective material forming a first blend;
   processing the first blend to form a dry second blend comprising prolactin in at least one protective layer, wherein said protective layer is specifically designed to degrade as a response to change in an environmental trigger, and
   adding the dry second blend to said nutritional food or nutritional feed or drink.

The preparing a protected prolactin may further comprise:
forming a round core from the second blend;
drying the core;
collecting the dehydrated core;
making a first suspension, comprising the dehydrated core in a liquid second protective material;
drying the first suspension in a fluidized bed drier;
collecting the fluidized bed -dried suspension;
making a second suspension, comprising the collected dried suspension in a liquid third protective material, and drying the second suspension in a fluidized bed drier.

Said first blend may be liquid.

Said first suspension may further comprise: a Maltodextrin, a vitamin, an antioxidant, a protease inhibitor, a growth hormone, an EGF (Epidermal Growth Factor), an insulin and insulin-like growth factor, an insulin-like growth factor's binding protein, an immunoglobulin, a proline-rich polypeptide, a lactoferrin, a protease, a lactalbumin, an interleukin, a lysozyme, a TGFA (Transforming Growth Factor A), a PDGF (Platelet Derived Growth Factor) or combination thereof.

Second suspension may further comprise: a maltodextrin, a vitamin, an antioxidant, a protease inhibitor, a growth hormone, an EGF (Epidermal Growth Factor), an insulin and insulin-like growth factor, an insulin-like growth factor's binding protein, an immunoglobulins, a proline-rich polypeptide, a lactoferrin, a protease, a lactalbumin, an interleukin, a lysozyme, a TGFA (Transforming Growth Factor A), a PDGF (Platelet Derived Growth Factor) or combination thereof.

In the supplemented nutritional food or feed or drink manufacture, maltodextrin may have a dextrose equivalent (DE) between 2 and 64, preferably 18..

Prolactin may be derivatized ex- vivo. Said derivatization may be done by enzymatic digestion, physical methods, chemical methods, or any combination thereof.

The supplemented nutritional food or feed or drink manufacture may further comprise an agglomeration step.

Said agglomeration step may result in particle average diameter between about 0.1 and about 5,000 micrometers.

The core may be inert.

Forming the round core may further comprise:
flash freezing said liquid blend;
collecting the droplets produced;
lyophilizing the collected droplets;
collecting the lyophilized droplets, thereby creating a round core.

Said core may include prolactin.

Said third protective material may be designed to thermally protect prolactin for no less than 2 minutes at a temperature of no less than 95°C.

Said second protective material may be designed to protect said bioactive compound from proteolytic enzymes and pH of no more than 4.75.

### DETAILED DESCRIPTION OF THE INVENTION

Suckling in humans and other mammals has multiple beneficial effects on infants' well being, including optimal nutrition, protection against a wide range of infection related diseases and promoting small intestine growth and development.

In suckling rats only 16 % of the total plasma prolactin originates from mother's milk (Gonella et al., 1989; Whitworth and Grosvenor, 1995). Nevertheless, we have realized an importance in supplementing the prolactin self-produced by babies with prolactin from exogenous sources such as formulas for preparation for bottle-feeding.

Accordingly, a nutritional composition for a subject is disclosed, the composition comprising microencapsulated prolactin.

Encapsulations that are considered suitable for such nutritional compositions have been described in full in WO05/115473, the disclosure in its entirety is cited for the purpose of indicating the background of embodiments of the invention and illustrating the state of the art.

The term "prolactins" includes fragments and/or derivatives of prolactin and/or compounds that are similar or analogous to prolactin found in a natural food sources such as natural unprocessed milk, unprocessed eggs, plant material, animal tissue, recombinant DNA technology, or the result of PCR, or the result of chemical synthesis.

The terms "analogous" or "analog" or "analogue" interchangeably refer to a structure that is similar in function to one in another kind of organism but is of dissimilar evolutionary origin.

According to one aspect, prolactin which is analogous to milk and eggs prolactin or prolactin variants, is used as supplements for human infant foods and animal infant feeds.

In some embodiments, the mammal is a preterm human infant, term human infant, a human baby, human toddler, human adolescent, human adult or human old person.

The mammal may also be a grown or mature animal.

Prolactin may be supplemented to food, feed or drink at levels measured in human mother's milk, i.e. between about 40 and 800 mU/L; alternatively, prolactin may be supplemented to promote health or growth in infants. Levels of prolactin in the latter preparations for human infants are preferably at levels of 100 to 600 mU/L.

Preferably, the prolactin variants in the nutritional composition are either similar, or identical, to the variants present in natural unprocessed colostrum, a full milk or egg. Most preferably, variants are selected that are specifically suitable for specific mammals. In particular, preferably the prolactin variants in a nutritional composition for feeding a certain animal are similar or identical to those in the milk or colostrum of the same animal.

Optionally, all of the variants present in the colostrum, full milk or egg are included in the nutritional composition. Alternatively, the prolactin variants in the nutritional compositions are essentially non-glycosylated and/or non-phosphorylated and/or non-dimerized or polymerized.

The ratio between of the variants present in natural unprocessed colostrum, full milk or egg may approximately be the ratio of the same variants included in the nutritional composition. In an alternative embodiment the proportion of non-phosphorylated and non-glycosylated prolactin is higher than the proportion of these variants in unprocessed colostrum, full milk or egg.

The release of the prolactin into the nutritional composition or directly to the subject receiving the nutritional compositions can be following exposure to an environmental trigger.

The term "trigger" refers to a change in environmental conditions sufficient to initiate degradation in the encapsulating materials of the encapsulating layers used in the composition and methods, the change leading to release of the bioactive, viable compounds encapsulated therein. The reference environmental condition may be time, or temperature, or moisture content, or pressure, or pH, or ionic strength, or enzymatic activity, or a combination thereof.

The environmental condition change may be selected as either a change of over ±2.5% in the reference environmental condition, or over ±5%, ±10, ±15%, ±20% , ±25%, ±30%, ±35%, ±40%, ±45% or ±50%.

In one embodiment, the protective layer surrounding or incorporating prolactin is specifically designed to degrade, or in another embodiment, undergo controlled release, as a response to exposure to the change in environmental condition, which is in another embodiment time, or in another embodiment temperature, or in another embodiment moisture content, or in another embodiment pressure, or in another embodiment pH, or in another embodiment ionic strength, or in another embodiment enzymatic activity, or in another embodiment a combination thereof.

Therefore, a core wherein prolactin is embedded, may be coated with a protective material to protect the active core from digestion in a digestive system of a subject, and release the core which in another embodiment, releases the active compound, only as a response to an increase in pH.

The active core, which may be encapsulated in an encapsulating material allowing release of the core based on increase in pH, is further encapsulated with another encapsulating material, designed to protect the core from increased temperature as described herein.

The skilled person in the art would recognize that the order of environmental triggers releasing prolactin is not rigid and depending on the environmental conditions of manufacturing, environmental conditions of integration into food or feed products, environmental conditions of storage after integration onto food or feed products, desired delivery location within the gastrointestinal system, timing and physiological activity desired, the encapsulating layers could accommodate those requirements without departing from the scope of the invention as described herein.

Any factor, which may affect the entrapment of the subject prolactin in a biodegradable matrix, and thereby affect its initial loading, or subsequent release, or a combination thereof, may be utilized according to the disclosed methods and compositions.. Such factors may comprise inter- alia, the initial solvent concentration, its molecular size and polarity, the temperature and pressure under which the solvent is removed, molecular weight number (MWn) average of the biodegradable matrix, its polydispersity index, the size and polarity of the prolactin, when the biodegradable matrix is a polymer, the monomer ratio and distribution along the copolymer's chain, or a combination thereof.

In addition, D/L ratio within each monomer of a biodegradable polymer will affect release rates. The term D/L ratio may refer to the ratio of monomer molecules that affect the direction (D-right, L-left), in which a cross- polarized lens will be rotated when observing a single optically active monomer, like lactic acid. Since most mammals have D-specific enzymes, that ratio will affect the digestion rate of the biodegradable biopolymer, affecting its molecular weight and consequently its viscosity, thereby affecting release rate of any entrapped prolactin.

Complexes between the prolactin and the protective layer may be formed via covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. Complexation may affect the compound's solubility in aqueous solution, eliminate aggregation, enhance the physical and/or chemical stability of the compound, change the immunogenicity or reactivity of the compound, promote gut maturation, small intestinal growth and/or functional maturation, enhance calcium absorption across intestinal epithelial membranes, stimulate mitosis in T lymphocytes or combination thereof.

The compositions may also serve to increase the rate of weight gain, improve the FCR (Feed Conversion Ratio) of newborn animals, reduce the mortality rate of newborn animals, prevent diarrhea or other gastric disorders or increase the life expectancy of newborn animals after birth.

Also disclosed is a method for preparing an encapsulated prolactin in a nutritional food or feed, comprising mixing the prolactin with an appropriate encapsulating material in liquid form forming a blend, then processing the blend formed to form a functionally multilayered protected dry blend, wherein the protective layer is specifically designed to degrade as a response to change in an environmental trigger, and then adding the dry blend to the nutritional food or feed, thereby preparing a multilayered encapsulation of the prolactin in a nutritional feed.

Also disclosed is a method for the encapsulation of a prolactin, comprising; (i) mixing a prolactin with a wall-forming encapsulating material, and (ii) rapidly cooling the wall forming material thereby resulting in encapsulation of the prolactin.

The abovementioned process may produce a core of a matrix entrapping the prolactin, alternatively, the core does not initially contain a prolactin and is therefore inert. The core produced may be substantially rounded, to improve the addition of additional encapsulating layers.

Forming the round core may further comprise flash freezing said liquid blend, collecting the droplets produced, lyophilizing the droplets collected and collecting the lyophilized droplets, thereby creating a round core, wherein said core may comprise a prolactin.

Encapsulation may refer to the process where one or more prolactin, prolactin variant and/or prolactin analog are coated with, or entrapped within, another food grade or feed grade or pharmaceutical grade material or matrix. Encapsulation of heat sensitive compounds, such as for example nutraceutical components, enzymes or bioactive proteins, into matrixes that are edible and digestible, is generally difficult for a number of reasons. Conventional encapsulation processes, which expose matrix material and encapsulants to high temperatures and moisture such as those encountered in pelleting and extrusion, causes thermal destruction or loss of biological viability of the encapsulant. Thus, either large initial load of encapsulant, a very expensive and potentially hazardous preposition, would be required, or the encapsulant would not stand the encapsulation process at all. If the encapsulant can be encapsulated into a matrix under sufficiently low temperatures, the resulting product is a solid that is characterized as a hard glass-like solid that is capable of being processed further to yield a flowable powder, amenable to additional processing.

The temperature at which the particles are consumed, or the eating temperature, may generally be lower than 50 degrees Celsius, which is far below the glass transition temperature, Tg. Careful design of the glassy matrix can release the prolactin under desired conditions of temperature, moisture, pH or enzymatic environment.

The encapsulated matrix could be used as dense pellets for a variety of processing applications, where a controlled release of the heat sensitive encapsulant is desired. The physical hardness of the products and their mechanical stability are advantageous for many processing applications.

The encapsulant may be food grade, or feed grade. The encapsulant may be a polysaccharide, or a maltodextrin, or milk powder, or a whey protein, or a lipid, or a gum, or a cellulose, or an amorphous lactose, or a combination thereof.

Mixing the prolactin with an appropriate encapsulating material forming a blend may further comprise mixing said compound with an encapsulant.

Also disclosed is a method of manufacture of a protected prolactin to retain biological activity of the protein.

The invention may be used to preserve biological activity of a prolactin from adverse temperature, or from adverse pressure, adverse humidity, adverse pH, adverse osmotic concentration, adverse ionic concentration, adverse enzymatic degradation, chemical degradation, presence of metals, surfactants and/or chelators, radiation (including UV, IR, or visible light or combination thereof), microbial degradation or from physical changes including first or second order phase transitions.

The term "first order phase transition" may refer to a discontinuity in the first derivative of Gibbs free energy with temperature at a constant concentration [(<3G/δT)c]. Alternatively, the term "first order phase transition" refers to crystallization, or to condensation, or to evaporation, or to melting.

The term "second order phase transition" may refer to a discontinuity in the second derivative of Gibbs free energy with temperature at a constant concentration [i.e (95G/δT)c= (9H/δT)c]. Alternatively, the term "second order phase transition" refers to glass/rubber transition, or to onset of rotational mobility (β-transition), or to onset of vibrational mobility, or to antemelting.

An analogue to the protected prolactin may be present in a natural mammalian milk or natural eggs, but its concentration is significantly lower, non viable, non available or non-existent in commercially processed human infant foods or animal infant feeds.

"Mammal" for purposes of treatment refers to any animal classified as a mammal, including humans, domestic and farm animals, and zoo, sports, or pet animals, such as dogs, horses, cats, hamsters, rats, mice, cattle, pigs, goats, sheep, etc. Preferably, the mammal is human.

Concentration as used herein refers to Molar concentration and its fractions, or percentage relative to that existing in colostrum, full milk and eggs.

The term "significantly lower" refers to the amount of the compound analogue to the prolactin in commercially processed milk being between about 0.01 to about 50 percent of that present in natural unprocessed colostrum, full milk or egg.

The encapsulating material may be food grade, or the encapsulating material may be feed grade, or the encapsulating material may be pharmaceutical grade, or a combination thereto.

Also disclosed is a method for preparing at least one encapsulated prolactin in a nutritional food formulation or in a nutritional feed formulation, comprising mixing the prolactin with an appropriate encapsulating material forming a blend, then processing the blend formed to form a functionally multilayered protected dry blend, wherein each of the protective layers is specifically designed to degrade as a response to change in an environmental trigger and then adding the dry blend to the nutritional food formulation or nutritional feed formulation, wherein the processing of the blend further comprises the forming of a round or non-round core, followed by drying of the core in a fluidized bed dryer, collecting the dehydrated core, suspending the dehydrated blend in a second functional encapsulating liquid, drying the suspension in a fluidized bed dryer and collecting the dehydrated suspension followed by resuspending the suspension obtained in the previous step in a third functional encapsulating fluid, then drying the resuspension a fluidized bed and finally adding the dry blend obtained to the nutritional food formulation or nutritional feed formulation, thereby preparing a multilayered encapsulation of a prolactin in a nutritional food formulation or a nutritional feed formulation. The initial blend may be liquid.

A second protective layer, or a third protective layer, or a fourth protective layer, or a fifth protective layer, or a sixth protective layer, or a seventh protective layer, or an eighth protective layer, or a ninth protective layer, or a tenth protective layer may further comprise a functional encapsulating material such as a maltodextrin, or a vitamin, or an antioxidant, or a protease inhibitor, or a growth hormone, or an EGF (Epidermal Growth Factor), or an insulin and insulin-like growth factor, or an insulin-like growth factor's binding protein, or an immunoglobulin, or a proline-rich polypeptide, or a lactoferrin, or a protease, or a lactalbumin, or an interleukin, or a lysozyme, a TGFA (Transforming Growth Factor A), or a PDGF (Platelet Derived Growth Factor) or combination thereof.

The second, or the third functional encapsulating material or a fourth functional encapsulating material, or a fifth functional encapsulating material, or a sixth functional encapsulating material, or a seventh functional encapsulating material, or an eighth functional encapsulating material, or a ninth functional encapsulating material, or a tenth functional encapsulating material may be maltodextrin, which, may have a DE value between about 2 to about 64. The maltodextrin may have a DE of between about 2 and about 5, or between about 5 and about 10, or between about 10 and about 15, or between about 15 and about 20, or between about 20 and about 25, or between about 25 and about 30, or between about 30 and about 35, or between about 35 and about 40, or between about 45 and about 50, or between about 50 and about 55, or between about 55 and about 60, or between about 60 and about 64. The maltodextrin may have a DE of 18. Alternatively, the maltodextrin has a DE of 6.

A protecting layer enables the maintenance of the bioactive properties of the prolactin while in a "dormant state", which may refer to the period when the protected prolactin is dehydrated, such as those present in powdered infant formulas, milk substitute products, and semi-solid / solid mixes and pellets. The term "dormant state" of the prolactin may also refer to the preservation of the native tertiary and quaternary structures of the prolactin in an anhydrous state.

The protecting layer provides protection to the encapsulated prolactin, so that the prolactin shall maintain its bioactive properties in hostile conditions such as high temperatures normally leading to the proteins' denaturation, or high pressures, or humidity, or adverse osmotic pressures, or high or low pH, or strong enzymatic degradation, or high solvent concentration and the like, or a combination of at least two of the above.

Based on a triggering event, an outer protection layer may be dissolved, or outer protection layers are dissolved, and the "dormant" prolactin will be released and become physiologically active.

Preferably, the release of prolactin is essentially while the microcapsules are in contact with different parts of the gastrointestinal tract. The release may mainly be in the small intestine.

The encapsulated prolactin will be protected from conditions encountered during commercial pelleting and extrusion processes, including but not limited to cold pelleting and extrusion or hot pelleting extrusion, either at standard temperatures and pressures or at conditions different than standard temperatures and pressures.

The encapsulated prolactin will be protected from conditions encountered during commercial size reduction processes, including processing by colloid mills, both stator rotor of the frusto conical type, as well as crown and tooth type, ball mills, impact mills, jet impingement mills, homogenizing mills, sonication, high velocity mixers or membrane emulsification devices.

The encapsulated prolactin will be protected from conditions encountered during commercial baking processes, or in another embodiment freezing processes.

The external functional encapsulating material in the external encapsulating layer can be designed to thermally protect the prolactin for no less than 2 minutes at a temperature of no less than 95°C. The external functional encapsulating material in the external encapsulating layer may also be designed to thermally protect the prolactin for no less than 1 minute at a temperature of no less than 120°C. The external functional encapsulating material may also be designed to protect the prolactin from photolytic enzymes and pH of no more than 4.75. The external functional encapsulating material may also be designed to protect the prolactin from any combination of factors as described hereinabove.

Also disclosed is a method for supplementing a nutritional food or feed or drink of a mammal, comprising incorporating a nutritional composition for a subject, comprising a prolactin analogous to one found in a natural food source, and a protective layer, wherein release of the prolactin into the subject is in another embodiment the result of an environmental event, in said nutritional food or feed or drink, thereby supplementing said food or feed or drink.

Therefore, a newborn formulation may be provided, comprising a prolactin being encapsulated or embedded in a multilayered edible ingredient, which protects and preserves the prolactin making it viable in the newborn.

The newborn formulation may be an infant formula or a milk replacement / substitute or semi-solid feed or solid feed for mammal's newborn consumption.

The term "milk replacer / substitute" may refer to any milk replacer / substitute for mammalian neonates wherein the mammals are of the human, bovine, equine, and swine families for examples calf, lamb, pig, cows, sheep, goat, yaez, cats, dogs and horses. In one embodiment, the milk replacer / substitute refers to any milk replacer / substitute, suitable for mammalian neonates, wherein the mammals are of the feline and canine families.

The plasticized material may be carbohydrate polysaccharides, such as pentosans, or a physically or chemically modified starch or cyclodextrin or mixtures thereof.

The plasticized material may be a polymer such as polyvinylpyrrolidone (PVP, Povidone) or other non-hydrophobic polymers such as N-vinylpyrrolidone (NVP) and poly(vinyl)acetate copolymers, (polyvinyl)alcohol chitosan or mixtures thereof. The plasticized material may be cellulose esters, cellulose ethers, and polyethylene glycol. The plasticized material may be a hydrocolloid such as xanthan, carragenan, alginate, gum arabic, gum acacia, gum tragacanth, gum conjac or mixtures thereof.

The plasticized material may be glutenins or gliadins, such as vital wheat gluten or isolated gluten, or zein, or vegetable or proteins such as protein from soy or milk, or mixtures thereof.

Starches that can be used may be physically or chemically modified starches, with amylose/amylopectin ratios of between about 1 to about 0.001, derived from corn, wheat, rice, or potato, tapioca, yuka, arrow root or a combination thereof.

Sources of starch which may be used also include flours from cereals such as corn, wheat, durum wheat, rice, barley, oat, rye or mixtures thereof.

The wall material used may be poly (DL- lactide-co-glycolide).

The food-grade or feed-grade encapsulating material, used in the neonate formulation may comprise polysaccharide, maltodextrin, milk powder, whey protein, lipid, gum, cellulose or combinations thereof.

The newborn formulation may comprise approximately uniformly sized particles of encapsulated plant-extracted prolactin, wherein the particles have a diameter between about 0.1 and about 5,000 micrometers. Preferably, D3,2 is the area average particle diameter.

The formulation can be used for post weaning mammals. Post-weaning mammals as used herein refer to the age at which the intensively grown mammals are typically weaned off the mother's milk. For example, intensively grown lambs are typically weaned between 25 - 35 days from birth. Intensively grown piglets are typically weaned between 18 - 30 days from birth; Intestively-grown calves are typically weaned between 40 - 70 days from birth.

In all of these newborn animals, the provided quantity of the milk replacer containing the prolactin may be gradually reduced, and the quantity of the prolactin in mix, pellets or other semi-solid or solid feed may be gradually increased.

The integration of the prolactin in mix / pellets / drink is advantageous for as long as 1-9 months post-birth or post-weaning. Alternatively, the prolactin is beneficial for either 1-2 months, 2-3 months, 3-4 months, 4-5 months, 5-6 months, 6-7 months, 7-8 months, or 8-9 months post-birth or post-weaning.

The solid or semi-solid feed formulation may be in the form a mash, or pellets, or granules, or agglomerate, or extrude or combinations thereof.

The embedded or encapsulated prolactin maintains or substantially may maintain its biological function during the digestion of the food or feed.

The embedded or encapsulated prolactin may be released upon contact with a liquid.

The newborn animal solid or semi-solid feed formulation may comprise uniformly sized particles of an encapsulated prolactin, wherein the particles have an average size of between about 10 to about 4000 micrometers.

Products containing protected prolactin are consumed by a variety of subjects such as preterm infants, post-discharge preterm infants, term infants, babies, toddlers, children, adolescents, adults, elderly humans, or infants or adults of non-human animals, such as bovine, porcine, caprine, feline, canine, equine species or infants or adults of any other non-human animals.

Formulas and milk replacers for preterm infants, specially preterm infants born between weeks 24 - 36, where such formulas or milk replacers contain a protected or un - protected prolactin, may be supplemented with a protected or non-protected bioactive protein prior to consumption, are used to assist in accelerating the development or maturation of the preterm infant's gastrointestinal tract or prevent or reduce the incidence of frequently fatal diseases associated with premature birth, such as NEC (Necrotizing Enterocolitis).

Foods and drinks of preterm or term infants may incorporate a protected prolactin or unprotected prolactin, when provided immediately or shortly after birth, assist in eliminating or reducing the onset of autoimmune diseases such as IDDM, Celiac, Inflammatory Bowel Disease, and Crohn's Disease etc.

A protected prolactin may be premixed and packaged in a separate package from the food or feed or drink, or prior to consumption by a subject, the package containing the protected prolactin is opened, and the protected prolactin is incorporated into the food or feed or drink of a subject, thus creating a bioactive supplemented food or feed or drink of a subject.

A method for encapsulating and embedding prolactin in mammalian newborn formulation is also disclosed, comprising the steps of, (i) mixing the prolactin with an edible food grade or feed grade or pharmaceutical grade encapsulating material forming a liquid blend; (ii) drying of the liquid blend; (iii) coating the dry blend with a additional food grade or feed grade or pharmaceutical grade encapsulating material layer; and (iv) adding the dry blend to the newborn formulation.

The mammalian newborn food formulation may be infant formula or milk replacer/substitute or other drink. Such a formulation may be a form of powder, a solution, a suspension, an emulsion, an ointment, a cream in both liquid, semi-solid or a solid form.

A formulation for post weaning mammals which is a solid or a semi-solid formulation is also disclosed, comprising an encapsulated and embedded prolactin prepared by the following process: (i) mixing the compound with a food grade or feed grade or pharmaceutical grade encapsulating material so as to form a liquid blend; (ii) drying of the liquid blend so as to form a dry blend; (iii) coating the dry blend with a additional food grade or feed grade or pharmaceutical grade encapsulating material layer; and (iv) adding the dry blend to the mammalian solid or semi-solid feed formulation. The solid or semi-solid formulation may be in a form of pellets or mash / mix.

Further, the step of mixing the prolactin and the wall forming food grade or feed grade or pharmaceutical grade material, may involve the addition of liquid, such as, but not limited to: water, saline, alcohol, molasses, organic solvents or similar food grade or feed grade or pharmaceutical grade encapsulating material solvent.

The ratio between the food grade or feed grade or pharmaceutical grade material and the solvent of the food grade or feed grade or pharmaceutical grade encapsulating material may be between about 1:1 to about 1:1,000, or between 1:3 and 1:100.

The dry blend may undergo further size- reduction.

The encapsulated prolactin may be further encapsulated by an additional protection layer, which may be formed of the same food grade or feed grade or pharmaceutical grade encapsulating material or, a different food or feed grade or pharmaceutical grade encapsulating material.

The role of the protective layer is to protect the core from adverse environmental conditions such as temperature, steam and/or pressure, or other environmental triggers. Alternatively or additionally, the protective layer's role is to protect the core from degradation.

Each combination of a different number and type of encapsulation layers result in a unique product suitable for the protection of the prolactin during encapsulation manufacturing conditions, and/or during integration of prolactin into food or feed or drink products and/or during storage, and for maturation of the gastrointestinal system, and/or for properties and characteristics of the subject at the specific age it is being fed. Accordingly, a multi-layer encapsulation for a piglet of 2 days old may substantially differ from multi-layer encapsulation required for a 25 days old piglet.

The dry blend may be further mixed with said food or feed grade or pharmaceutical grade encapsulating material so as to form another layer of food grade or feed grade or pharmaceutical grade encapsulating material layer enveloping the prolactin.

The food grade or feed grade or pharmaceutical grade encapsulating material may be a polysaccharide, milk powder, whey protein, lipid, gum Arabic microcrystalline cellulose, their analogs or combinations thereof.
The food grade or feed grade or pharmaceutical grade encapsulating material may be a solid at temperatures of up to 85°C.

The step of drying the food grade or feed grade or pharmaceutical grade encapsulating material and prolactin may be done using the methods including but not limited to: freeze drying, vacuum drying, spray drying, osmotic dehydration, fluidized bed dehydration, solvent evaporation dehydration, sonication assisted dehydration, microwave-assisted dehydration, RF-assisted dehydration, either alone or commercially acceptable combinations thereof.

The liquid mix may be lyophilized after incorporating a prolactin and a food grade or feed grade or pharmaceutical grade encapsulating material ingredient.

Lyophilization produces particles containing a protected prolactin and a food grade or feed grade or a pharmaceutical grade encapsulating material in a glassy matrix.

A flash freezer may be employed to dry the liquid mix through the utilization of liquid gas, which may be nitrogen, or CO₂, or propane, or any suitable compressible refrigerant gas.

The size of the droplets may vary between about 10 and about 5,000 micrometers.

The droplets size distribution depends on a variety of parameters such as freeze sprayer nozzle size, liquid gas temperature, chamber temperature, mix components ratio, mix and gas flow rates, encapsulating material concentration, plasticizer type or freeze chamber wall geometry.

The size distribution of the glassy droplets resulting from the process may range between 50 microns and 1,000 microns.

This treatment results in glassy frozen micro droplets, where each micro droplet contains a protected prolactin, a food grade or feed grade or pharmaceutical grade encapsulating material and the food grade or feed grade or pharmaceutical grade solvent.

Once such frozen droplets are placed in temperatures above the melting temperature of the mix, the liquid mix from the previous phase of the process shall be reconstituted.

The process may further include the freeze- drying of a combination of prolactin and a food grade or feed grade or pharmaceutical grade encapsulating material.

Freeze drying may be carried out on either a liquid mixture of prolactin and a food grade or feed grade or pharmaceutical grade encapsulating material or on frozen glassy micro droplets as described hereinabove.

The result of this freeze drying process is dry glassy material which includes a food grade or feed grade or pharmaceutical grade encapsulating material and the prolactin.

Freeze drying may be performed on a liquid mixture, the result of the process being bulk dry material, porous by nature, containing a glassy matrix of the dried food-grade or feed grade or pharmaceutical grade encapsulating material encapsulating the prolactin.

Freeze-drying may be performed on the output of the flash freeze spraying process, resulting in glassy droplets, with the food grade or feed grade or pharmaceutical grade encapsulating material incorporating the prolactin.

Low-temperature spray drying of combination of prolactin and a food grade or feed grade or pharmaceutical grade encapsulating material may be carried out.

The prolactin may be dispersed in the food grade or feed grade or pharmaceutical grade encapsulating material and atomized at a maximum temperature of 45°C.

The maximum temperature may be 37°C, preventing denaturation of the prolactin.

Spray drying may be carried out on a liquid mixture of a protected prolactin, a food grade or feed grade or pharmaceutical grade encapsulating material and a chaperon-like protecting protein, resulting in dry material which comprises the food grade or feed grade or pharmaceutical grade encapsulating material and prolactin.

The dehydration of the food grade or feed grade or pharmaceutical grade encapsulating material and prolactin may be conducted at a temperature, which is preferably below the denaturation temperature of prolactin.

The dehydration of the food grade or feed grade or pharmaceutical grade encapsulating material and the prolactin may be carried out at a temperature below the onset temperature for prolactin's denaturation threshold or degradation threshold.

Preferably, the dehydration of compositions of the encapsulating material and variants of prolactin is carried out at a temperature below the lowest onset temperature of said variant's denaturation threshold or degradation threshold.

The dehydration process of the food grade or feed grade or pharmaceutical grade encapsulating material and the prolactin may be carried out at a maximum temperature of 50°C.

The step of drying the liquid blend may result in glassy freeze-dried droplets containing prolactin and a food grade or feed grade or pharmaceutical grade encapsulating material.

The step of freeze-drying may be preceded by a step of spraying the liquid blend through an atomizer in the presence of a liquid gas.

Extrusion may be used as an encapsulation method in which a core material is dispersed in a liquid mass of prolactin and a food grade or feed grade or pharmaceutical grade encapsulating material and ultimately formed into microcapsule.

Encapsulating or embedding protected prolactin in the formulation described above may involve an additional step of premixing the blend in a small volume of the newborn formulation or food grade or feed grade or pharmaceutical grade encapsulating material, or semi solid or solid formulation, to ensure homogeneity prior to its mixing with the whole formulation.

Protection processes suited for use as used herein include, but are not limited to those which produce protected prolactin in the form of a: powder, a micro-encapsulated powder, a nano-encapsulated powder, a liquid, a micro-emulsified liquid, a nano-emulsified liquid, a solution, a micro-emulsified solution, a nano-emulsified solution, a spread, a mash, an ointment, micro droplets, nano-droplets, tablets and solids such as for example, pellets.

The encapsulation process may include duplex, W/O/W, O/W/O, double or multiple emulsions.

A mix of prolactin and a food grade or feed grade or pharmaceutical grade encapsulating material and a surfactant selected from the group of surfactants having an HLB value substantially below 7 may be suspended in a non-miscible, food grade or feed grade or pharmaceutical grade material and further mixed, affecting size reduction using methods hereinabove mentioned.

The milled emulsion may further be mixed with a food grade or feed grade or pharmaceutical grade material that is miscible with the encapsulating material, and a food grade or feed grade or pharmaceutical grade surfactant selected from the group of surfactants having an HLB value substantially higher than 7 and further reduced in size using one of the methods hereinabove mentioned.

Following formulation of a prolactin, micro emulsification or nano emulsification of the formulated prolactin may be conducted.

The formulated prolactin may be mixed with an emulsion incorporating water, oil phase and surfactant. As a result of such mixing, the prolactin molecules are reorganized into the dispersed phase of the emulsion.

The protection provided to prolactin by the micro emulsion or nano emulsion may relate to temperature exposure protection, and improved solubility of the prolactin within the food or feed with which it is integrated, following the release of the prolactin from its encapsulation prior to its consumption and/or during the digestion process.

The prolactin in the nano emulsion or micro emulsion is initially protected within the liquid micro emulsion or liquid nano emulsion.

A method for the encapsulation of prolactin in a food grade or feed grade or pharmaceutical grade glassy matrix is also disclosed, the method comprising; (i) mixing a homogeneous intimate mixture between a prolactin and a wall forming, food grade or feed grade or pharmaceutical grade encapsulating material creating a blend, (ii) mixing said blend with an appropriate plasticizer, (iii) rapidly removing said plasticizer while inhibiting crystallization of the wall forming material thereby resulting in encapsulation of the prolactin in a food grade or feed grade or pharmaceutical grade glassy matrix.

A method for the encapsulation of a prolactin is also disclosed, comprising; (i) mixing a prolactin with a molten wall-forming food grade or feed grade or pharmaceutical grade encapsulating material, and (ii) rapidly cooling the molten, a wall forming material thereby resulting in encapsulation of the prolactin in a food-grade or feed-grade or pharmaceutical-grade glassy matrix.

"glassy-state matrix" refers to an amorphous metastable solid wherein rapid removal of a plasticizer causes increase in viscosity of the biopolymer to the point where translational mobility of the critical polymer segment length is arrested and alignment corresponding to the polymer's inherent adiabatic expansion coefficient is discontinued.

Hydrophilic materials both of a monomer and a polymeric nature either exist as or can be converted into amorphous states which exhibit the glass/rubber transitions characteristic of amorphous macromolecules. These materials have well defined glass transition temperatures Tg which may depend on the molecular weight or on the molecular complexity of the glass forming substance. Tg is depressed by the addition of diluents. Water is the universal plasticizer for all such hydrophilic materials. Therefore, the glass/rubber transition temperature may be adjustable by the addition of water or an aqueous solution, or alternatively by the removal of water or an aqueous solution.

The plasticizer may be any substance of molecular weight lower than that of the biocompatible polymer that creates an increase in the free interstitial volume. The plasticizer may be an organic compound, which is triglyceride of varying chain length, or water.

A method for encapsulating and embedding a prolactin in newborn formulation is also disclosed, the method comprising; (i) mixing prolactin with a liquid food grade or feed grade or pharmaceutical grade encapsulating material so as to form a liquid blend, (ii) drying of the liquid blend so as to form a dry blend; (iii) coating the dry blend with an additional layer comprised of a food grade or feed grade or pharmaceutical grade encapsulating material, where each such layer has different properties relating to environmental conditions in regard of durability and degradation, and (iv) adding the dry blend to the newborn formulation, thereby being a method for encapsulating and embedding a prolactin in newborn formulations.

A newborn formulation is disclosed comprising a prolactin being encapsulated or embedded in a food grade or feed grade or pharmaceutical grade encapsulating material.

A method for encapsulating or embedding a prolactin in newborn solid or semi solid feed formulation or newborn drink is also disclosed, comprising the steps of; (i) mixing the prolactin with a liquid food grade or feed grade or pharmaceutical grade encapsulating material so as to form a liquid blend, (ii) drying of the liquid blend so as to form a dry blend, (iii) coating the dry blend with a additional layer comprised of a food grade or feed grade or pharmaceutical grade encapsulating material, where each such layer has different durability and degradation properties relating to environmental conditions, and (iv) adding the dry blend to the newborn animal solid or semi solid feed formulation of newborn drink, thereby being a method for encapsulating and embedding the prolactin in newborn animal solid or semi solid feed formulation or newborn drink.

A newborn animal solid or semi-solid feed formulation or newborn drink is also disclosed, comprising a prolactin being encapsulated or embedded in a food grade or feed grade or pharmaceutical grade material.

The following examples are presented in order to more fully illustrate some embodiment of the invention. They should, in no way be construed, however, as limiting the scope of the invention.

### EXAMPLE 1 - Fluidized Bed Coating Process specifications

### Coating samples

PMDP - Polycose® (core) + [MD (maltodextrin)+ prolactin] (coating solution) -→(one concentration - 2 IU/gr)
[LMDP - Lactose (core) + [MD + prolactin] (coating solution) - > (one concentration - 2 IU/gr)
MMDP - Maltodextrin (core) + [MD + prolactin] (coating solution) - > (one concentration -2 IU/gr ; MD 18 concentrations of 10%, 20%, 30% MD 18 + Vitamin C 10% (one concentration on each core and MD 18 coating)

### Coating conditions

The mixing is done under food grade regulation conditions and with compliance with the Biodar ISO9001:2000 quality system procedures. Throughout the manufacturing process the product temperature does not exceed 37°C. The process is performed at a slow rate to prevent agglomeration.

### Sampling

From each stage in the process a sample of 10 grams is taken, packed in a bag and labeled to indicate the sample number.

### EXAMPLE 2 - Prolactin solution premix preparation

### Mixing conditions

Mixing is done under cGMP conditions and with compliance with HACCP procedures

### Solution preparation

A Maltodextrin DE-18, prolactin and saline 0.45% solution is prepared using 20% Maltodextrin DE-18, prolactin (100 IU/ml) at a ratio of 10cc to 500gr active ingredient coated core (MD/Polycose core coated with MD + prolactin layer). Saline 0.45% is added to complete to a 100% solution. Saline is added partially to the solution. The rest of the Saline solution is used to rinse the prolactin bottles to ensure all material has been washed out and added to the solution. [The solution is mixed until the Maltodextrin is completely dissolved.

### EXAMPLE 3 - In Vitro testing

Several in-vitro tests are performed on the prolactin product in order to verify that the manufacturing process does not adversely affect the required product characteristics and bioactivity, and further to ensure that it consistently meets its technical specifications.

### Osmolarity testing

The Osmolarity testing should indicate that the addition of prolactin microcapsule powder to the RTF formula has no appreciable effect on the final solution osmolarity, thus the formula remaining within its specifications. The RTF (Ready-To-Feed) liquid formula has a defined osmolarity that is important for suitable nutrients consumption. Therefore, a test is performed to verify that the addition of prolactin microcapsule powder to the RTF does not change the osmolarity of the liquid formula. The test is performed by immersing 1.0g to 1.5g of prolactin microcapsule powder in 60ml preterm RTF formula bottle, analyzing the osmolarity and comparing it to a control containing the same RTF formula without prolactin. Each sample is analyzed in triplicates.

### Product stability in liquid infant formula over time

The prolactin microcapsule powder is intended to be consumed immediately after solubilization in the infant formula. Nevertheless, the prolactin stability over time is measured by adding a pre-defined quantity of liquid prolactin (concentration of 100µU per aliquot) to 60ml Materna^{™} preterm infants formula in a baby bottle, and the prolactin quantity is analyzed immediately following addition, then after 3, 6, 9, 12, 15, 18, 21 and 24 hours by using the Elisa kit. Furthermore, in order to evaluate the final product homogeneity, at each time interval, sampling is taken at the upper, middle and lower layers of the sampled RTF bottle, as well as after formula liquid stirring.

### Prolactin microcapsule powder stability at extreme temperatures

Since prolactin is a temperature sensitive protein, the product ability to protect the prolactin component following exposure of the encapsulated powder to high temperature for various durations, is measured.

It should be clear that the description of the embodiments and attached Figures set forth in this specification serves only for a better understanding of the invention, without limiting its scope as covered by the following Claims.

## Claims

1. Use of prolactin for the manufacture of a nutritional composition for enhancing small intestinal growth, small intestinal functional maturation and/or calcium absorption across intestinal epithelial membranes enhancing gut maturation, and/or stimulating mitosis in T lymphocytes, the composition comprising prolactin identical or similar or analogous to prolactin found in a natural food source microencapsulated in at least one protective layer, wherein release of said prolactin from the protective layer in said subject is the result of an environmental trigger.

2. The use of claim 1, wherein the natural food source is selected from the group consisting of natural unprocessed milk, natural unprocessed eggs, animal tissue, colostrum and serum,
or/preferably wherein the prolactin comprises at least one prolactin variant, each variant being an analogue of a naturally occurring prolactin or a functional derivative thereof;
or/preferably, wherein the subject is a mammal, preferably wherein the mammal is a human, or
specifically formulated for said subject.

3. The use of claim 2, wherein the subject is a mammal and the at least one variant in the composition being homologous to a variant of the mammal.

4. The use of claim 3,
wherein all variants in the composition are homologous to a variant in the mammal; or
wherein the at least one variant in the composition is identical to a variant in the mammal, preferably wherein all of the variants in the composition are identical to a variant in the mammal.

5. The use of any one of claims 3 or 4, wherein the mammal is a human, preferably
wherein the human is selected from the group consisting of a neonate, a preterm human infant, a term human infant, a baby, a toddler, an adolescent, an adult and an old person.

6. The use of any one of the preceding claims, wherein the prolactin comprises a 23 kDa form.

7. The use of any one of claims 2 to 6, wherein the prolactin comprises at least one prolactin variant, each variant being an analogue of a naturally occurring prolactin or a functional derivative thereof, wherein the at least one variant is selected from the group consisting of non-glycosylated prolactin, non-phosphorylated prolactin, prolactin monomer and any combination thereof.

8. The use of any one of the preceding claims,
wherein the composition comprises at least one complex between the prolactin and the at least one protective layer, preferably wherein the composition further comprises one or more water-soluble polymer selected from the group polyethylene glycol, copolymers of polyethylene glycol and polypropylene glycol, carboxymethyl cellulose, dextran, polyvinyl alcohol, polyvinylpyrrolidone and polyproline, for complexation between the prolactin and at least one protective layer via covalent attachment of the water-soluble polymers to both the prolactin and the protective layer; preferably
wherein the protective layer is specifically designed to degrade as a response to the environmental trigger, wherein said environmental trigger is a change in at least one of temperature, moisture content, pressure, pH, ionic strength, enzymatic activity, time passed since providing the subject with the nutritional composition, or any combination thereof, preferably wherein the protective layer is selected from the group consisting of polysaccharide, maltodextrin, milk powder, whey protein, lipid, gum, celluloses, amorphous lactose, or combinations thereof.

9. Use of prolactin for the manufacture of a supplemented nutritional food or feed or drink for enhancing small intestinal growth, small intestinal functional maturation and/or calcium absorption across intestinal epithelial membranes enhancing gut maturation, and/or stimulating mitosis in T lymphocytes, the supplemented nutritional food comprising nutritional food and a prolactin composition, the prolactin composition comprising prolactin identical or similar or analogous to prolactin found in a natural food source microencapsulated in at least one protective layer, wherein release of said prolactin from the at least one protective layer in said subject is the result of an environmental trigger.

10. The use of claim 9,
wherein the manufacture comprises adding the prolactin composition to the nutritional food or feed or drink;
or/preferably wherein the manufacture comprises:
a. preparing a protected prolactin comprising mixing said prolactin with an appropriate first protective material forming a first blend;
b. processing the first blend to form a dry second blend comprising prolactin in at least one protective layer, wherein said protective layer is specifically designed to degrade as a response to change in an environmental trigger; and
c. adding the dry second blend to said nutritional food or nutritional feed or drink, preferably wherein preparing a protected prolactin further comprises:
a. forming a round core from the second blend;
b. drying the core;
c. collecting the dehydrated core;
d. making a first suspension, comprising the dehydrated core in a liquid second protective material;
e. drying the first suspension in a fluidized bed drier;
f. collecting the fluidized bed -dried suspension;
g. making a second suspension, comprising the collected dried suspension in a liquid third protective material; and
h. drying the second suspension in a fluidized bed drier.

11. The use of claim 10,
wherein the first blend is liquid; or
wherein the first suspension further comprises at least one of a Maltodextrin, a vitamin, an antioxidant, a protease inhibitor, a growth hormone, an EGF (Epidermal Growth Factor), an insulin and insulin- like growth factor, an insulin-like growth factor's binding protein, an immunoglobulin, a proline-rich polypeptide, a lactoferrin, a protease, a lactalbumin, an interleukin, a lysozyme, a TGFA (Transforming Growth Factor A), a PDGF (Platelet Derived Growth Factor) or a combination thereof; or
wherein the second suspension further comprises at least one of a maltodextrin, a vitamin, an antioxidant, a protease inhibitor, a growth hormone, an EGF (Epidermal Growth Factor), an insulin and insulin- like growth factor, an insulin-iike growth factor's binding protein, an immunoglobulins, a proline-rich polypeptide, a lactoferrin, a protease, a lactalbumin, an interleukin, a lysozyme, a TGFA (Transforming Growth Factor A), a PDGF (Platelet Derived Growth Factor) or a combination thereof.

12. The use of claim 11, wherein the maltodextrin has a dextrose equivalent (DE) between 2 and 64, preferably wherein the dextrose equivalent is 18.

13. The use of any one of claims 9 to 12,
wherein prolactin is derivatized ex- vivo, preferably wherein the derivatization is done by at least one of enzymatic digestion, physical methods, chemical methods, or any combination thereof; or/preferably
further comprising an agglomeration step, preferably wherein the agglomeration step results in particle average diameter between about 0.1 and about 5,000 micrometers.

14. The use of claim 10, wherein preparing a protected prolactin further comprises:
a. forming a round core from the second blend;
b. drying the core;
c. collecting the dehydrated core;
d. making a first suspension, comprising the dehydrated core in a liquid second protective material;
e. drying the first suspension in a fluidized bed drier;
f. collecting the fluidized bed -dried suspension;
g. making a second suspension, comprising the collected dried suspension in a liquid third protective material; and
h. drying the second suspension in a fluidized bed drier.
and
wherein the core is inert; or
wherein forming the round core further comprises:
a. flash freezing said liquid blend;
b. collecting the droplets produced;
c. lyophilizing the collected droplets; and
d. collecting the lyophilized droplets, thereby creating a round core; or
wherein the core comprises prolactin; or
wherein the third protective material is designed to thermally protect prolactin for at least 2 minutes at a temperature of no less than 95°C; or
wherein the second protective material is designed to protect said prolactin from proteolytic enzymes and pH of no more than 4.75.

## Patentansprüche

1. Verwendung von Prolactin zur Herstellung einer Nahrungsmittelzusammensetzung zur Steigerung des Wachstums des Dünndarms, zur funktionellen Reifung des Dünndarms und/oder zur Calcium-Absorption über die Darmepithel-Membranen zur Steigerung der Darmreifung, und/oder zur Stimulierung der Mitose in T-Lymphozyten, worin die Zusammensetzung Prolactin enthält, das identisch oder analog ist zu in einer natürlichen Nahrungsquelle vorkommendendem Prolactin, das in mindestens einer schützenden Schicht mikroverkapselt ist, wobei die Freisetzung des Prolactins aus der Schutzschicht in dem Individuum das Ergebnis einer auslösenden Umweltbedingung ist.

2. Verwendung nach Anspruch 1, worin die natürliche Nahrungsmittelquelle ausgewählt ist aus der Gruppe bestehend aus natürlicher, nicht-verarbeiteter Milch, natürlichen nichtverarbeiteten Eiern, Tiergewebe, Kolostrum und Serum,
oder/vorzugsweise worin das Prolactin mindestens eine Prolactin-Variante umfasst, worin jede Variante ein Analog eines natürlich vorkommenden Prolactins oder eines funktionellen Derivats davon ist;
oder/vorzugsweise worin das Individuum ein Säuger ist, vorzugsweise worin der Säuger ein Mensch ist, oder
spezifisch für das Individuum formuliert.

3. Verwendung nach Anspruch 2, worin das Individuum ein Säuger ist und die mindestens eine Variante in der Zusammensetzung homolog zu einer Variante des Säugers ist.

4. Verwendung nach Anspruch 3,
worin alle Varianten in der Zusammensetzung homolog zu einer Variante in dem Säuger sind; oder
worin die mindestens eine Variante in der Zusammensetzung identisch ist zu einer Variante in dem Säuger, vorzugsweise worin alle Varianten in der Zusammensetzung identisch sind zu einer Variante in dem Säuger.

5. Verwendung nach einem der Ansprüche 3 oder 4, worin der Säuger ein Mensch ist, vorzugsweise worin der Mensch ausgewählt ist aus der Gruppe bestehend aus einem Neugeborenen, einem menschlichen Frühgeborenen, einem termingerechten menschlichen Säugling, einem Baby, einem Kleinkind, einem Heranwachsenden, einem Erwachsenen und einer alten Person.

6. Verwendung nach einem der vorstehenden Ansprüche, worin das Prolactin eine 23 kDa Form umfasst.

7. Verwendung nach einem der Ansprüche 2 bis 6, worin das Prolactin mindestens eine Prolactin-Variante umfasst, worin jede Variante ein Analog eines natürlich vorkommenden Prolactins oder eines funktionellen Derivats davon ist, worin die mindestens eine Variante ausgewählt ist aus der Gruppe bestehend aus einem nicht-glycosylierten Prolactin, nicht-phosphorylierten Prolactin, Prolactin-Monomer und jeder Kombination davon.

8. Verwendung nach einem der vorstehenden Ansprüche,
worin der Zusammensetzung mindestens einen Komplex zwischen dem Prolactin und der mindestens einen Schutzschicht umfasst, vorzugsweise worin die Zusammensetzung weiter ein oder mehrere Wasser-lösliche Polymer(e) umfasst, ausgewählt aus der Gruppe bestehend aus Polyethyleneglycol, Copolymeren von Polyethyleneglycol und Polypropylenglycol, Carboxymethylcellulose, Dextran, Polyvinylalkohol, Polyvinylpyrrolidon und Polyprolin, zur Komplexbildung zwischen dem Prolactin und der mindestens einen Schutzschicht über kovalente Bindung des Wasser-löslichen Polymers an das Prolactin und die Schutzschicht; vorzugsweise
worin die Schutzschicht spezifisch ausgestaltet ist, sich in Antwort auf eine Umweltbedingung zu zersetzen, worin die Umweltbedingung eine Änderung mindestens eines der Temperatur, des Feuchtigkeitsgehalts, Drucks, pH's, der Ionenstärke, der enzymatischen Aktivität, der Zeitspanne nach Verabreichen der Nahrungsmittelzusammensetzung an das Individuum oder jede Kombination davon ist, vorzugsweise worin die Schutzschicht ausgewählt ist aus der Gruppe bestehend aus Polysaccharid, Maltodextrin, Milchpulver, Molkenprotein, Fett, Gum, Cellulosen, amorpher Lactose oder Kombinationen davon.

9. Verwendung von Prolactin zur Herstellung eines ergänzten Nahrungsmittels oder ergänzten Nahrungsmitteln oder Getränks zur Steigerung des Wachstums des Dünndarms, zur funktionellen Reifung des Dünndarms und/oder zur Calcium-Absorption über die Darmepithel-Membranen zur Steigerung der Darmreifung, und/oder zur Stimulierung der Mitose in T-Lymphozyten, worin das ergänzte Nahrungsmittel ein Nahrungsmittel und eine Prolactin- Zusammensetzung enthält, das/die Prolactin enthält, das identisch oder ähnlich ist zu in einer natürlichen Nahrungsquelle vorkommendenden Prolactin und in mindestens einer schützenden Schicht mikroverkapselt ist, wobei die Freisetzung des Prolactins aus der Schutzschicht in dem Individuum das Ergebnis einer auslösenden Umweltbedingung ist.

10. Verwendung nach Anspruch 9,
worin die Herstellung umfasst, Zugeben der Prolactin-Zusammensetzung zu dem Nahrungsmittel oder den Nahrungsmitteln oder dem Getränk;
oder/vorzugsweise worin die Herstellung umfasst;
a. Herstellen von geschütztern Prolactin, welches umfasst, Mischen des Prolactins mit einem geeigneten ersten Schutzmaterial unter Bildung einer ersten Mischung;
b. Verarbeiten der ersten Mischung unter Bildung einer trockenen zweiten Mischung, welches Prolactin in mindestens einer Schutzschicht umfasst, worin die Schutzschicht spezifisch ausgestaltet ist, sich als Antwort auf eine Änderung einer Umweltbedingung zu zersetzen, und
c. Zusetzen der zweiten trockenen Mischung zu dem Nahrungsmittel oder den Nahrungsmitteln oder dem Getränk,
vorzugsweise worin die Herstellung eines geschützten Prolactins weiter umfasst:
a. Bilden eines runden Kerns aus der zweiten Mischung;
b. Trocknen des Kerns;
c. Gewinnen des dehydrierten Kerns;
d. Herstellen einer ersten Suspension, welche den dehydrierten Kern in einer Flüssigkeit eines zweiten schützenden Materials enthält;
e. Trocknen der ersten Suspension in einem Flüssigbett-Trockner;
f. Gewinnen der im Flüssigbett getrockneten Suspension;
g. Herstellen einer zweiten Suspension, welchen die gewonnene getrocknete Suspension in einem dritten Schutzmaterial umfasst; und
h. Trocknen der zweiten Suspension in einem Flüssigbett-Trockner.

11. Verwendung nach Anspruch 10,
worin die erste Mischung flüssig ist; oder
worin die erste Suspension weiter umfasst, mindestens eines von Maltodextrin, einem Vitamin, einem Antioxidationsmittel, einem Proteaseinhibitor, einem Wachstumsfaktor, einem EGF (Epidermis Wachstumsfaktor), einem Insulin und Insulin-ähnlichen Wachstumsfaktor, einem Insulin-ähnlichen Wachstumsfaktor-Bindungsprotein, einem Immunoglobulin, einem Prolin-reichen Polypeptid, einem Lactoferrin, einer Protease, einem Lactalbumin, einem Interleukin, einem Lysozym, einem TGFA (Transforming Wachstumsfaktor A), einem PDGF (Platelet abgeleiteten Wachstumsfaktor) oder einer Kombination davon; oder
worin die zweite Suspension weiter umfasst, mindestens eines von Maltodextrin, einem Vitamin, einem Antioxidationsmittel, einem Proteaseinhibitor, einem Wachstumsfaktor-Hormon, einem EGF (Epidermis-Wachstumsfaktor), einem Insulin und Insulin-ähnlichen Wachstumsfaktor, einem Insulin-ähnlichen Wachstumsfaktor-Bindungsprotein, einem Immunglobulin, einem Prolin-reichen Polypeptid, einem Lactoferrin, einer Protease, einem Lactalbumin, einem Interleukin, einem Lysozyme, einem TGFA (Transformierender Wachstumsfaktor A), einem PDGF (Platelet abgeleiteten Wachstumsfaktor) oder einer Kombination davon.

12. Verwendung nach Anspruch 11, worin das Maltodextrin ein Dextrose-Equivalent (DE) zwischen 2 und 64 aufweist, vorzugsweise worin das Dextrose-Equivalent 18 ist.

13. Verwendung nach einem der Ansprüche 9 bis 12,
worin das Prolactin ex-vivo derivatisiert wurde, vorzugsweise worin die Derivatisierung erfolgt durch mindestens eines von enzymatischem Verdau, körperlichen Verfahren, chemischen Verfahren oder jeder Kombination davon; oder/vorzugsweise
welche weiter einen Agglomerations-Schritt umfasst, vorzugsweise worin der Agglomerations-Schritt zu einem mittleren Teilchendurchmesser zwischen etwa 0,1 und etwa 5,000 Mikrometer führt.

14. Verwendung nach Anspruch 10, worin die Herstellung eines geschützten Prolactins weiter umfasst:
a. Bilden eines runden Kerns aus der zweiten Mischung;
b. Trocknen des Kerns;
c. Gewinnen des dehydrierten Kerns;
d. Herstellen einer ersten Suspension, welche den dehydrierten Kern in einem zweiten flüssigen Schutzmaterial enthält;
e. Trocknen der ersten Suspension in einem Flüssigbett-Trockner;
f. Gewinnen der in einem Flüssigbett-Trockner getrockneten Suspension;
g. Herstellen einer zweiten Suspension, welche die gewonnene getrocknete Suspension in einem flüssigen dritten Schutzmaterial enthält; und
h. Trocknen der zweiten Suspension in einem Flüssigbett-Trockner; und
worin der Kern inert ist; oder
worin Bilden des runden Kerns weiter umfasst:
a. Schnellgefrieren der flüssigen Mischung;
b. Gewinnen der hergestellten Tröpfchen;
c. Lyophilisieren der gewonnenen Tröpfchen; und
d. Gewinnen der lyophilisierten Tröpfchen, wodurch ein runder Kern hergestellt wird; oder
worin der Kern Prolactin umfasst; oder
worin das dritte Schutzmaterial ausgestaltet ist Prolactin für mindestens 2 Minuten bei einer Temperatur von nicht unter 95°C zu schützen; oder
worin das zweite Schutzmaterial ausgestaltet ist, das Prolactin vor proteolytischen Enzymen und einem pH von nicht mehr als 4.75 zu schützen.

## Revendications

1. Utilisation de prolactine pour la fabrication d'une composition nutritionnelle pour améliorer la croissance de l'intestin grêle, la maturation fonctionnelle de l'intestin grêle et/ou l'absorption de calcium à travers les membranes épithéliales intestinales améliorant la maturation intestinale, et/ou pour stimuler la mitose dans des lymphocytes T, la composition comprenant de la prolactine identique ou similaire ou analogue à la prolactine trouvée dans une source alimentaire naturelle microencapsulée dans au moins une couche de protection, dans laquelle la libération de ladite prolactine de la couche de protection chez ledit sujet est le résultat d'un déclencheur environnemental.

2. Utilisation selon la revendication 1, dans laquelle la source alimentaire naturelle est choisie dans le groupe constitué de lait naturel non traité, oeufs naturels non traités, tissu animal, colostrum et sérum,
ou/de préférence, dans laquelle la prolactine comprend au moins une variante de prolactine, chaque variante étant un analogue d'une prolactine présente naturellement ou un dérivé fonctionnel de celui-ci ;
ou/de préférence, dans laquelle le sujet est un mammifère, de préférence dans laquelle le mammifère est un humain, ou
spécifiquement formulée pour ledit sujet.

3. Utilisation selon la revendication 2, dans laquelle le sujet est un mammifère et ladite au moins une variante dans la composition étant homologue à une variante du mammifère.

4. Utilisation selon la revendication 3,
dans laquelle toutes les variantes dans la composition sont homologues à une variante chez le mammifère ; ou
dans laquelle ladite au moins une variante dans la composition est identique à une variante chez le mammifère, de préférence dans laquelle toutes les variantes dans la composition sont identiques à une variante chez le mammifère.

5. Utilisation selon l'une quelconque des revendications 3 ou 4, dans laquelle le mammifère est un humain, de préférence dans laquelle l'humain est choisi dans le groupe constitué d'un nouveau-né, un nouveau-né humain prématuré, un nouveau-né humain à terme, un bébé, un bambin, un adolescent, un adulte et une personne âgée.

6. Utilisation selon l'une quelconque des revendications précédentes, dans laquelle la prolactine comprend une forme de 23 kDa.

7. Utilisation selon l'une quelconque des revendications 2 à 6, dans laquelle la prolactine comprend au moins une variante de prolactine, chaque variante étant un analogue d'une prolactine présente naturellement ou un dérivé fonctionnel de celui-ci, dans laquelle ladite au moins une variante est choisie dans le groupe constitué de prolactine non glycosylée, prolactine non phosphorylée, monomère de prolactine et n'importe quelle combinaison de ceux-ci.

8. Utilisation selon l'une quelconque des revendications précédentes,
dans laquelle la composition comprend au moins un complexe entre la prolactine et ladite au moins une couche de protection, de préférence dans laquelle la composition comprend en outre un ou plusieurs polymères hydrosolubles choisis parmi le groupe de polyéthylène glycol, copolymères de polyéthylène glycol et polypropylène glycol, carboxyméthylcellulose, dextrane, alcool polyvinylique, polyvinylpyrrolidone et polyproline, pour une complexation entre la prolactine et ladite au moins une couche de protection par l'intermédiaire d'un attachement covalent des polymères hydrosolubles à la fois à la prolactine et à la couche de protection ; de préférence
dans laquelle la couche de protection est spécifiquement conçue pour se dégrader en tant que réponse au déclencheur environnemental, dans laquelle ledit déclencheur environnemental est un changement dans au moins un paramètre parmi la température, la teneur en humidité, la pression, le pH, la force ionique, l'activité enzymatique, le temps écoulé depuis que l'on a fourni au sujet la composition nutritionnelle, ou n'importe quelle combinaison de ceux-ci, de préférence dans laquelle la couche de protection est choisie dans le groupe constitué de polysaccharide, maltodextrine, lait en poudre, protéine de lactosérum, lipide, gomme, celluloses, lactose amorphe, ou leurs combinaisons.

9. Utilisation de prolactine pour la fabrication d'un aliment ou alimentation animale ou boisson nutritionnel, complété, pour améliorer la croissance de l'intestin grêle, la maturation fonctionnelle de l'intestin grêle et/ou l'absorption de calcium à travers les membranes épithéliales intestinales améliorant la maturation intestinale, et/ou stimuler la mitose dans des lymphocytes T, l'aliment nutritionnel complété comprenant l'aliment nutritionnel et une composition de prolactine, la composition de prolactine comprenant de la prolactine identique ou similaire ou analogue à la prolactine trouvée dans une source alimentaire naturelle microencapsulée dans au moins une couche de protection, dans laquelle la libération de ladite prolactine de ladite au moins une couche de protection chez ledit sujet est le résultat d'un déclencheur environnemental.

10. Utilisation selon la revendication 9,
dans laquelle la fabrication comprend l'ajout de la composition de prolactine à l'aliment ou alimentation animale ou boisson nutritionnel ;
ou/de préférence, dans laquelle la fabrication comprend :
a. la préparation d'une prolactine protégée comprenant le mélange de ladite prolactine avec un premier matériau de protection approprié en formant un premier mélange ;
b. le traitement du premier mélange pour former un second mélange sec comprenant de la prolactine dans au moins une couche de protection, dans laquelle ladite couche de protection est spécifiquement conçue pour dégrader en tant que réponse à un changement dans un déclencheur environnemental ; et
c. l'ajout du second mélange sec audit aliment nutritionnel ou à ladite alimentation animale ou boisson nutritionnelle, de préférence dans laquelle la préparation d'une prolactine protégée comprend en outre :
a. la formation d'un noyau rond à partir du second mélange ;
b. le séchage du noyau ;
c. le recueil du noyau déshydraté ;
d. la fabrication d'une première suspension, comprenant le noyau déshydraté dans un deuxième matériau de protection liquide ;
e. le séchage de la première suspension dans un séchoir à lit fluidisé ;
f. le recueil de la suspension séchée en lit fluidisé ;
g. la fabrication d'une seconde suspension, comprenant la suspension séchée recueillie dans un troisième matériau de protection liquide ; et
h. le séchage de la seconde suspension dans un séchoir à lit fluidisé.

11. Utilisation selon la revendication 10,
dans laquelle le premier mélange est liquide ; ou
dans laquelle la première suspension comprend en outre au moins l'un parmi une maltodextrine, une vitamine, un antioxydant, un inhibiteur de protéase, une hormone de croissance, un facteur de croissance épidermique (EGF), un facteur de croissance d'insuline et analogue à l'insuline, une protéine de liaison de facteur de croissance analogue à l'insuline, une immunoglobuline, un polypeptide riche en proline, une lactoferrine, une protéase, une lactalbumine, une interleukine, un lysozyme, un facteur de croissance transformant A (TGFA), un facteur de croissance dérivé de plaquettes (PDGF) ou une de leurs combinaisons ; ou
dans laquelle la seconde suspension comprend en outre au moins l'un parmi une maltodextrine, une vitamine, un antioxydant, un inhibiteur de protéase, une hormone de croissance, un facteur de croissance épidermique (EGF), un facteur de croissance d'insuline et analogue à l'insuline, une protéine de liaison de facteur de croissance analogue à l'insuline, une immunoglobuline, un polypeptide riche en proline, une lactoferrine, une protéase, une lactalbumine, une interleukine, un lysozyme, un facteur de croissance transformant A (TGFA), un facteur de croissance dérivé de plaquettes (PDGF) ou une de leurs combinaisons.

12. Utilisation selon la revendication 11, dans laquelle la maltodextrine possède un équivalent dextrose (DE) compris entre 2 et 64, de préférence dans laquelle l'équivalent dextrose vaut 18.

13. Utilisation selon l'une quelconque des revendications 9 à 12,
dans laquelle la prolactine est dérivatisée ex vivo, de préférence dans laquelle la dérivatisation est faite par au moins un parmi une digestion enzymatique, des procédés physiques, des procédés chimiques, ou n'importe quelle combinaison de ceux-ci ; ou/de préférence
comprenant en outre une étape d'agglomération, de préférence dans laquelle l'étape d'agglomération entraîne un diamètre particulaire moyen compris entre environ 0,1 et environ 5000 micromètres.

14. Utilisation selon la revendication 10, dans laquelle la préparation d'une prolactine protégée comprend en outre :
a. la formation d'un noyau rond à partir du second mélange ;
b. le séchage du noyau ;
c. le recueil du noyau déshydraté ;
d. la fabrication d'une première suspension, comprenant le noyau déshydraté dans un deuxième matériau de protection liquide ;
e. le séchage de la première suspension dans un séchoir à lit fluidisé ;
f. le recueil de la suspension séchée en lit fluidisé ;
g. la fabrication d'une seconde suspension, comprenant la suspension séchée recueillie dans un troisième matériau de protection liquide ; et
h. le séchage de la seconde suspension dans un séchoir à lit fluidisé.
et
dans laquelle le noyau est inerte ; ou
dans laquelle la formation du noyau rond comprend en outre :
a. la congélation instantanée dudit mélange liquide ;
b. le recueil des gouttelettes produites ;
c. la lyophilisation des gouttelettes recueillies ; et
d. le recueil des gouttelettes lyophilisées, ce qui crée un noyau rond ; ou
dans laquelle le noyau comprend de la prolactine ; ou
dans laquelle le troisième matériau de protection est conçu pour protéger thermiquement la prolactine pendant au moins 2 minutes à une température n'étant pas inférieure à 95 °C ; ou
dans laquelle le deuxième matériau de protection est conçu pour protéger ladite prolactine vis-à-vis d'enzymes protéolytiques et d'un pH n'excédant pas 4,75.
